# EUROPÄISCHE PATENTSCHRIFT

(11) **EP 4 618 942 B1**
(45) Veröffentlichungstag und Bekanntmachung des Hinweises auf die Patenterteilung: **09.09.2026**
(21) Anmeldenummer: 23786562.1
(22) Anmeldetag: 10.10.2023
(51) Int. Cl.: A61K 8/37, A61K 8/88, A61P 17/00, A61Q 19/00

(54) **ANTIMIKROBIELLE ZUBEREITUNG UMFASSEND EIN POLYLYSIN UND PROPANEDIOL CAPRYLATE**
ANTIMICROBIAL COMPOSITION COMPRISING POLYLYSINE AND PROPANEDIOL CAPRYLATE
COMPOSITION ANTI-MICROBIENNE COMPRENANT DE LA POLYLYSINE ET DU PROPANEDIOL CAPRYLATE

(30) Priorität: 15.11.2022 DE 102022212098
(43) Veröffentlichungstag der Anmeldung: 24.09.2025
(73) Patentinhaber: Beiersdorf AG, 22529 Hamburg (DE)
(72) Erfinder: TRILLER, Benjamin, 22941 Jersbek (DE); RÜBENHAUS, Philipp, 22399 Hamburg (DE)
(74) Vertreter: Beiersdorf AG
(86) Internationale Anmeldenummer: PCT/EP2023/077988
(87) Internationale Veröffentlichungsnummer: WO 2024/104652

(56) Entgegenhaltungen:
- WO-A1-2020/197669
- CN-A- 110 215 461
- GENRICH FLORIAN ET AL: "Propanediol Caprylate: Shifting the Microbiome, Sebum Levels and Classic Approach to Dandruff", COSMETICS&TOILETRIES, 1 June 2021 (2021-06-01), XP093056860, Retrieved from the Internet <URL:https://www.cosmeticsandtoiletries.com/testing/article/21836364/symrise-propanediol-caprylate-shifting-the-microbiome-sebum-levels-and-classic-approach-to-dandruff> [retrieved on 20230622]

## Beschreibung

Die Erfindung betrifft eine dermatologischen und/oder kosmetischen Zubereitung umfassend ein Polylysin und Propanediol Caprylate mit antimikrobieller Wirkung gegen *Propionibacterium acnes.*

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht. Einen wesentlichen Anteil an einem schönen und attraktiven Äußeren haben dabei der Zustand und das Aussehen der Haut.

Bei fettig-öliger und unreiner Haut, als welche man den Übergangszustand zwischen der gesunden normalen und der krankhaft veränderten Aknehaut bezeichnet, produziert die Haut erhöhte Mengen an Talg (Seborrhoe). Dieser dient zahlreichen Mikroorganismen, insbesondere *Propionibacterium acnes* und *Pityrosporum-Arten* als idealer Nährboden. Die Mikroorganismen zersetzen den Talg zu Glycerin und Fettsäuren, wodurch die Talgdrüsen zu erhöhter Produktion angeregt und die Follikelwandungen in der Haut angegriffen und zerstört werden. Dies ruft Entzündungen in der Haut (Pickel, Pusteln, Knoten, Zysten) hervor, welche oft nur narbig ausheilen, wodurch das optische Erscheinungsbild des an unreiner Haut leidenden Menschen dauerhaft geschädigt wird (W. Umbach [Hrsg], Kosmetik, Entwicklung, Herstellung und Anwendung kosmetischer Mittel, 2.Aufl. Thieme Verlag, Stuttgart, 1995).

Unter Akne (im engeren Sinne *acne vulgaris)* versteht man verschiedene Erkrankungen der Talgdrüsenfollikel, die durch Sekretions- und Verhornungsstörungen nachfolgende Entzündungen und eventuelle Vernarbung gekennzeichnet sind. Die *acne vulgaris* tritt vor hauptsächlich in der Pubertät auf und konzentriert sich in der Regel auf talgdrüsenreiche Hautbezirke (Gesicht, Nacken, Brust, Rücken). Durch Talgdrüsenhyperplasie und eine Verhornungsstörung der Follikel kommt es zu deren Verstopfung mit Bildung von Komedonen und den für *acne vulgaris* typischen Effloreszenzen (Pschyrembel, Klinisches Wörterbuch, 258. Aufl., Walter de Gruyter-Verlag, Berlin, 1998).

Zur Prophylaxe und Behandlung von Akne werden effektive Wirkstoffe benötigt, die die Besiedlung der Haut mit Mikroorganismen wie *Propionibacterium acnes* reduzieren bzw. verhindern. Somit tragen die Wirkstoffe nicht nur zu einem verbesserten Hautzustand sondern auch zu einem verbesserten Wohlbefinden des Anwenders bei.

Typische eingesetzte Wirkstoffe mit antimikrobieller Wirkung sind beispielsweise starke Oxidationsmittel, wie z.B. Benzoylperoxid; alpha-Hydroxysäuren, wie z.B. Milchsäure oder Salicylsäure; sowie aliphatische Dicarbonsäuren, wie z.B. Azelainsäure. Problematisch ist allerdings, dass die antimikrobielle Wirkung dieser Wirkstoffe gegenüber *Propionibacterium acnes* nur moderat ist. Somit müssen verhältnismäßig hohe Konzentrationen - Benzoylperoxid z.B. mit bis zu 5 Gew.-% und Azelainsäure mit bis zu 20 Gew.-% - in kosmetischen und dermatologischen Formulierungen eingesetzt werden. Bedingt durch diese hohen Konzentrationen wird nachteilig die Haut sehr stark strapaziert, was sich insbesondere in einer starken Austrocknung der Haut äußert. Ferner sind starke Hautirritationen möglich, die auf eine Reduktion des pH-Werts der Haut bei Anwendung der beschriebenen Wirkstoffe zurückzuführen ist.

Alternativ ist die topische Anwendung von effektiv gegen *Propionibacterium acnes* wirkenden Antibiotika, wie Clindamycin, Erythromycin und Tetracyclin, möglich. Insbesondere aufgrund des in der klinischen Praxis immer häufiger zu beobachtenden Auftretens resistenter *Propionibacterium acnes* Stämme sowie deren unspezifischem Wirkungsspektrum, welches zu einer starken Beeinträchtigung der gesunden humanen Mikroflora führt, geraten auch die in der Akne-Therapie geläufigen Antibiotika zunehmend ins Licht der Kritik.

Weiterhin sind dem Fachmann die Dokumente WO 2011117126 A2 und
WO 2002089791 A2 bekannt, welche die Wirkstoffe gegen *Propionibakterium acnes* offenbaren. Jedoch konnten diese Dokumente nicht auf die vorliegende Erfindung hindeuten.

CN 110215461 A offenbart eine Polylysin-haltige Zusammensetzung und deren Verwendung in der Behandlung von Akne.

Es verbleibt wünschenswert effektive antibiotikafreie Wirkstoffe oder Wirkstoffkombinationen bereitzustellen, die das Wachstum und die Verbreitung von Akne hervorrufenden Bakterien auf der Haut, insbesondere von *Propionibakterium acnes* unterdrückt und gleichzeitig wenig oder nicht Haut-irritierend wirken.

Überraschend für den Fachmann wurde nun gefunden, dass eine Wirkstoffkombination umfassend ein Polylysin und Propanediol Caprylate synergistische antimikrobielle Wirkung gegen *Propionibacterium acnes* aufweist.

Ein erster Gegenstand der Erfindung ist somit eine Wirkstoffkombination umfassend ein Polylysin und Propanediol Caprylate.

Auch Gegenstand der Erfindung ist eine kosmetische oder dermatologische Zubereitung umfassend die erfindungsgemäße Wirkstoffkombination.

Ein weiterer Aspekt der Erfindung ist die kosmetische Verwendung von der Wirkstoffkombination zur Reduzierung von *Propionibacterium acnes* auf der menschlichen Haut.

Ein weiterer Gegenstand der Erfindung ist die erfindungsgemäße Wirkstoffkombination zur Verwendung bei der Behandlung von Acne auf der menschlichen Haut.

Ferner ist Gegenstand der Erfindung die erfindungsgemäße Wirkstoffkombination zur Verwendung in der Prophylaxe und/oder Behandlung von Akne.

Wird in dieser Offenbarung der Ausdruck "gegen *Propionibakterium acnes"* verwendet, so bedeutet das, dass *Propionibacterium acnes* abgetötet wird und/oder das Wachstum und/oder die Ausbreitung des Bakteriums vermindert und/oder verhindert wird.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der Zubereitung. Sollten nachfolgend Verhältnisse von Komponenten/Substanzen/Stoffgruppen offenbart werden, so beziehen sich diese Verhältnisse auf Gewichtsverhältnisse der genannten Komponenten/ Substanzen/Stoffgruppen.

Werden nachfolgend Gewichtsprozentbereiche für die Bestandteile der Zubereitung angegeben, so umfasst die Offenbarung der vorliegenden Anmeldung ebenfalls alle Einzelwerte in Schritten von 0,1 Gew.-% innerhalb dieser Gewichtsprozentbereiche.

Die Formulierungen "erfindungsgemäß", "erfindungsgemäß vorteilhaft", "vorteilhaft im Sinne der vorliegenden Erfindung" etc. beziehen sich im Rahmen der vorliegenden Offenbarung immer sowohl auf die erfindungsgemäße Zubereitung sowie die erfindungsgemäße Verwendung und das Verfahren.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Der Begriff "frei von" bedeutet im Sinne der vorliegenden Offenbarung, dass der Anteil der genannten Substanz 0,1 Gew.-% nicht übersteigt und insbesondere 0 Gew.-%, bezogen auf das Gesamtgewicht der Zubereitung beträgt. Somit soll ein versehentliches Einschleppen durch Verunreinigungen nicht zum Ausschluss aus dem Schutzbereich führen.

Unter Emulgatoren werden alle Substanzen verstanden, welche im International Cosmetic Ingredient Dictionary and Handbook, Thirteenth Edition 2010, (ISBN 1-882621-47-6) unter der Bezeichnung "emulsifying agent" geführt werden. Unter Tensiden werden alle Substanzen verstanden, welche im International Cosmetic Ingredient Dictionary and Handbook, Thirteenth Edition 2010, (ISBN 1-882621-47-6) unter der Bezeichnung " surfactant " geführt werden.

Werden in dieser Offenbarung Viskositätswerte angegeben, so beziehen sich alle Werte auf eine Messung bei 25°C in einer 150 ml Weithalsflasche (VWR Nr.: 807-0001) mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr.1 (Artikelnr. 200 0191), geeignet für einen Viskositätsbereich bis 10.000 [mPa·s], Drehzahl Bereich 62,5 min-1, verwendet.

Werden in dieser Offenbarung Angaben zu einem Mittelwert gemacht, bezieht sich dieser Mittelwert immer auf arithmetische Mittel.

Werden in dieser Offenbarung Angaben zum Molekulargewicht von Polymeren gemacht, so beziehen diese sich auf eine Messung mittel Lichtstreuung.

Wird der Begriff Haut verwendet, so bezieht dieser sich bevorzugt auf die menschliche Haut.

Definitionsgemäß sind Polylysine Homopolymere von der Aminosäure Lysin. Die Vorläuferaminosäure Lysin enthält zwei Aminogruppen, eine am α-Kohlenstoff und eine am ε-Kohlenstoff. Beides kann der Ort der Polymerisation sein, was zu α-Polylysin oder ε-Polylysin führt. Polylysin ist ein Homopolypeptid aus der Gruppe der kationischen Polymere: Polylysin enthält bei pH 7 eine positiv geladene hydrophile Aminogruppe.

Vorteilhaft beträgt das Gewichtsverhältnis von Polylysin zu Propanediol Caprylate von 10:1 bis 1:10, bevorzugt von 5:1 bis 1:8, bevorzugt von 1:1 bis 1:5 und insbesondere bevorzugt von 1:2 bis 1:3.

Es ist erfindungsgemäß von Vorteil, wenn ε-Polylysin in der erfindungsgemäßen Wirkstoffkombination enthalten ist.

Vorteilhaft beträgt das Gewichtsverhältnis von ε-Polylysin zu Propanediol Caprylate von 10:1 bis 1:10, bevorzugt von 5:1 bis 1:8, bevorzugt von 1:1 bis 1:5 und insbesondere bevorzugt von 1:2 bis 1:3.

Bezogen auf die erfindungsgemäße Zubereitung ist es erfindungsgemäß bevorzugt, wenn der Anteil von Polylysin von 0,01 bis 1 Gew.-%, bevorzugt von 0,1 bis 0,5 Gew.-% und insbesondere bevorzugt von 0,15 bis 0,3 Gew.-% beträgt, jeweils bezogen auf die erfindungsgemäße Zubereitung.

Bezogen auf die erfindungsgemäße Zubereitung ist es erfindungsgemäß bevorzugt, wenn der Anteil von ε-Polylysin von 0,01 bis 1 Gew.-%, bevorzugt von 0,1 bis 0,5 Gew.-% und insbesondere bevorzugt von 0,15 bis 0,3 Gew.-% beträgt, jeweils bezogen auf die erfindungsgemäße Zubereitung.

Bezogen auf die erfindungsgemäße Zubereitung ist es erfindungsgemäß bevorzugt, wenn der Anteil von Propanediol Caprylate von 0,01 bis 3 Gew.-%, bevorzugt von 0,1 bis 1,5 Gew.-% und insbesondere bevorzugt von 0,2 bis 0,8 Gew.-% beträgt, jeweils bezogen auf die erfindungsgemäße Zubereitung.

Generell ist es insbesondere schwierig stabile Zubereitungen mit Polylysinen insbesondere ε-Polylysin bereitzustellen. Insbesondere sollen diese Zubereitungen nicht die Wirksamkeit der Wirkstoffkombination nicht verringern. Es konnten nun überraschend Zubereitungen bereitgestellt werden, welche die diese Anforderungen erfüllen.

Neben der Wirkstoffkombination umfassen die erfindungsgemäßen Zubereitungen vorteilhaft eine Ölphase und eine wässrige Phase.

Vorteilhaft beträgt der Anteil der flüssigen Öle in der Zubereitung von 5 bis 15 Gew.-%, bevorzugt von 6 bis 11 Gew.-% und insbesondere bevorzugt von 6,5 bis 10 Gew.-%, bezogen auf das Gesamtgewicht der Zubereitung.

Erfindungsgemäß vorteilhaft können als flüssige Öle natürliche und nicht natürliche Öle eingesetzt werden.

Unter natürlichen Ölen werden insbesondere vorteilhaft Öle pflanzlichen Ursprungs eingesetzt. Vorteilhaft enthaltene natürliche Öle sind gewählt aus der Gruppe Persea Gratissima Oil, Orbignya Oleifera Seed Oil, Argania Spinosa Kernel Oil, Prunus Armeniaca Kernel Oil, Simmondsia Chinensis Seed Oil, Butyrospermum Parkii Butter, Cocos Nucifera Oil, Silybum Marianum Seed Oil, Oenothera Biennis Oil, Olea Europaea Fruit Oil, Helianthus Annuus Seed Oil, Vitis Vinifera Seed Oil, Cannabis Sativa Seed Oil, Vegetable Oil, Gossypium Herbaceum Seed Oil, Arctium Lappa Seed Oil, Macadamia Ternifolia Seed Oil, Macadamia Integrifolia Seed Oil, Zea Mays Germ Oil, Prunus Amygdalus Dulcis Oil, Ricinus Communis Seed Oil, Brassica Campestris Seed Oil und/oder Glycine Soja Oil.

Sofern natürliche Öle enthalten sind, beträgt der Gesamtgehalt dieser Öle vorteilhaft von 1,0 bis 10 Gew.-%, bevorzugt 3,0 bis 8,0 Gew.-% und insbesondere bevorzugt von 4,0 bis 7,5 Gew.-%, bezogen auf das Gesamtgewicht der Zubereitung.

Weitere erfindungsgemäß vorteilhafte Öle sind ausgewählt aus den Anlagerungsprodukten von 6-60 Propylenoxid-Einheiten an ein- oder mehrwertige Alkanole mit einer Kettenlänge von 3 bis 22 C-Atomen. Diese werden im Sinne der Erfindung als Etheröle bezeichnet. Erfindungsgemäße Beispiele dieser Etheröle sind PPG-9 Butylether, PPG-12 Butylether, PPG-14 Butylether, PPG-15 Butylether, PPG-16 Butylether, PPG-17 Butylether, PPG-18 Butylether, PPG-2 Butylether, PPG-20 Butylether, PPG-22 Butylether, PPG-24 Butylether, PPG-26 Butylether, PPG-30 Butylether, PPG-33 Butylether, PPG-40 Butylether, PPG-52 Butylether, PPG-53 Butylether und PPG-15 Stearylether, sofern diese bei 20°C und 1013 hPa flüssig sind.

Weitere erfindungsgemäß vorteilhafte Öle sind ausgewählt aus der Gruppe der Ester aus gesättigten und/oder ungesättigten, verzweigten und/oder unverzweigten Alkancarbonsäuren einer Kettenlänge von 3 bis 17 C-Atomen und ungesättigten und/oder ungesättigten, verzweigten und/oder unverzweigten Alkoholen einer Kettenlänge von 3 bis 10 C-Atomen sowie aus der Gruppe der Ester aus aromatischen Carbonsäuren und ungesättigten und/oder ungesättigten, verzweigten und/oder unverzweigten Alkoholen einer Kettenlänge von 3 bis 15 C-Atomen, sofern das gewählte Esteröl bei 20°C flüssig ist. Solche Esteröle können vorteilhaft gewählt werden aus der Gruppe lsopropylmyristat, Isopropylpalmitat, Isopropylstearat, Isopropyloleat, 2-Ethylhexylpalmitat, 2-Ethylhexyllaurat, C12-15 Alkylbenzoat, sowie synthetische, halbsynthetische und natürliche Gemische solcher Ester, sofern das gewählte Esteröl bei 20°C und 1013 hPa flüssig ist.

Weiterhin ist es erfindungsgemäß von Vorteil, wenn als Öl Carylic/Capric Triglyceride in der Zubereitung enthalten ist. Sofern Carylic/Capric Triglyceride enthalten ist, beträgt der Anteil von Carylic/Capric Triglyceride vorteilhaft von 5,0 bis 10 Gew.-%, bevorzugt 5,5 bis 9 Gew.-% und insbesondere vorteilhaft von 6,0 bis 8,0 Gew.-%, jeweils bezogen auf das Gesamtgewicht der Zubereitung.

Weiterhin ist es erfindungsgemäß von Vorteil, wenn ein Emulgator enthalten ist, welcher keine Polyethylenglykol-Untereinheit aufweist. Eine Polyethylenglykoluntereinheit besteht mindestens aus 2 aufeinanderfolgenden Ethylenglykoleinheiten.

Vorteilhaft ist mindestens ein Emulgator enthalten, welcher ein Polyglycerylester ist. Unter Polyglycerylestern wird erfindungsgemäß ein Ester verstanden der aus Polyglycerin (Beispielsweise Polyglycerin-10) und mindestens einer Fettsäure gebildet wird.

Vorteilhaft sind die ein oder mehr weiteren Emulgatoren, gewählt aus der Gruppe Polyglyceryl-3 Methylglucose Distearate, Polyglyceryl-10 Caprate, Polyglyeryl-10 Caprylate, Polyglyceryl-10 Laurate, Polyglyceryl-10 Dilaurate, Polyglyceryl-10 Trilaurate, Polyglyceryl-10 Myristate, Polyglyceryl-10 Dimyristate, Polyglyceryl-10 Oleate, Polyglyceryl-10 Dioleate, Polyglyceryl-10 Stearate, Polyglyceryl-10 Distearate, Polyglyceryl-10 Diisostearate, Polyglyceryl-10 Isosteareate, Polyglyceryl-10 Tristearate, Polyglyceryl-6 Dicaprylate, Polygylceryl-6 Tricaprylate, Polyglyceryl-6 Caprylate, Polygylceryl-6 Dicaprate, Polyglyceryl-6 Laurate, Polyglyceryl-6 Trilaurate, Polyglyceryl-6 Oleate, Polyglyceryl-6 Ricinoleate, Polyglyceryl-6 Stearate, Polyglyceryl-5 Laurate, Polyglyceryl-5 Myristate, Polyglyceryl-5 Oleat, Polyglyceryl-5 Dioleat, Polyglyceryl-5 Stearate, Glyceryl Caprylate, Polyglyceryl-4 Laurate, Polyglyceryl-4 Caprate, Polyglyceryl-3 Cocoate, Polyglyceryl-3 Caprate/Caprylate/Succinate, Polyglyceryl-3 Laurate, Polygylceryl-2 Caprate, Polygylceryl-2 Caprylate und Mischungen davon.

Es ist insbesondere vorteilhaft, wenn als Polyglyceryl-10 Stearate enthalten ist.

Vorteilhaft beträgt der Anteil der Polyglycerylester von 0,01 bis 5 Gew.-%, bevorzugt von 0,5 bis 4 Gew.-% und insbesondere bevorzugt von 1,0 bis 2,5 Gew.-%, bezogen auf das Gesamtgewicht der Zubereitung.

Vorteilhaft beträgt der Anteil von Polyglyceryl-10 Stearate von 0,01 bis 5 Gew.-%, bevorzugt von 0,5 bis 4 Gew.-% und insbesondere bevorzugt von 1,0 bis 2,5 Gew.-%, bezogen auf das Gesamtgewicht der Zubereitung.

Weiterhin ist es vorteilhaft, wenn die Zubereitung mindestens einen Gelblildner gewählt aus den Polysacchariden. Bevorzugt Polysaccharide sind gewählt aus den unter den INCI Bezeichnung bekannten Materialien Xanthan Gum, Gellan Gum und Ceratonia Siliqua Gum. Insbesondere bevorzugt ist Ceratonia Siliqua Gum.

Sofern mindestens ein Polysaccharid enthalten ist, ist es bevorzugt, wenn der Gesamtanteil der Polysaccharide von 0,05 bis 3 Gew.-%, bevorzugt von 0,2 bis 1,5 Gew.-% und insbesondere bevorzugt von 0,3 Gew.-% bis 0,7 Gew.-% beträgt, jeweils bezogen auf das Gesamtgewicht der Zubereitung.

Sofern Xanthan Gum, Gellan Gum, und/oder Ceratonia Siliqua Gum enthalten sind, ist es bevorzugt, wenn der Gesamtanteil dieser Polysaccharide von 0,05 bis 3 Gew.-%, bevorzugt von 0,2 bis 1,5 Gew.-% und insbesondere bevorzugt von 0,3 Gew.-% bis 0,7 Gew.-% beträgt, jeweils bezogen auf das Gesamtgewicht der Zubereitung.

Sofern Ceratonia Siliqua Gum enthalten sind, ist es bevorzugt, wenn der Gesamtanteil von Ceratonia Siliqua Gum von 0,05 bis 3 Gew.-%, bevorzugt von 0,2 bis 1,5 Gew.-% und insbesondere bevorzugt von 0,3 Gew.-% bis 0,7 Gew.-% beträgt, jeweils bezogen auf das Gesamtgewicht der Zubereitung.

Weiterhin ist es vorteilhaft im Sinne der vorliegenden Erfindung, wenn die Zubereitung ein oder mehrere Fettalkohole aufweisend 14 bis 20 Kohlenstoffatome umfasst. Insbesondere bevorzugt sind Cetylalkohol, Stearylalkohol und/oder Mischungen dieser, welche unter der INCI Bezeichnung Cetearylalkohol bekannt sind. Sofern mindestens ein Fettalkohol aufweisend 14 bis 20 Kohlenstoffatome enthalten ist, so ist es vorteilhaft, wenn der Anteil dieser Fettalkohole von 1,0 bis 5 Gew.-%, bevorzugt von 1,5 bis 4,5 Gew.-% und insbesondere von 2,5 bis 4 Gew.-%, bezogen auf das Gesamtgewicht der Zubereitung, beträgt. Weiterhin ist es vorteilhaft, wenn der Gesamtanteil an Cetylalkohol, Stearylalkohol und/oder Cetearylalkohol von 1,0 bis 5 Gew.-%, bevorzugt von 1,5 bis 4,5 Gew.-% und insbesondere von 2,5 bis 4 Gew.-%, bezogen auf das Gesamtgewicht der Zubereitung, beträgt.

Weiterhin ist es vorteilhaft, wenn Ethanol in der Zubereitung enthalten ist, wobei es vorteilhaft ist, wenn der Gesamtanteil von Ethanol von 2 bis 10 Gew.-%, bevorzugt von 3 bis 8 Gew.-% und insbesondere bevorzugt von 4 bis 7 Gew.-%, bezogen auf das Gesamtgewicht beträgt.

Weiterhin ist es vorteilhaft, wenn die Zubereitung Phenoxyethanol, Ethylhexylgycerin, Methylpropanediol und/oder Caprylyl Glycol enthält. Sofern Phenoxyethanol enthalten ist, beträgt der Anteil von Phenoxyethanol vorteilhaft von 0,2 bis 0,9 Gew.-%, bezogen auf das Gesamtgewicht der Zubereitung. Sofern Ethylhexylgycerin enthalten ist, beträgt der Anteil von Ethylhexylgycerin vorteilhaft von 0,1 bis 0,5 Gew.-%, bezogen auf das Gesamtgewicht der Zubereitung. Sofern Methylpropanediol enthalten ist, beträgt der Anteil von Methylpropanediol vorteilhaft von 0,1 bis 0,9 Gew.-%, bezogen auf das Gesamtgewicht der Zubereitung. Sofern Caprylyl Glycol enthalten ist, beträgt der Anteil von Caprylyl Glycol vorteilhaft von 0,1 bis 0,9 Gew.-%, bezogen auf das Gesamtgewicht der Zubereitung.

Es ist ebenfalls vorteilhaft im Sinne der vorliegenden Erfindung, wenn die Zubereitung Glycerin umfasst. Sofern Glycerin enthalten ist, ist es vorteilhaft, wenn der Anteil von Glycerin von 2 bis 8 Gew.-%, bevorzugt von 3 bis 7 Gew.-% und insbesondere bevorzugt von 3,5 bis 6,5 Gew.-% beträgt, bezogen auf das Gesamtgewicht der Zubereitung.

Die Zubereitung der Erfindung ist ferner vorteilhaft dadurch gekennzeichnet, dass diese einen pH-Wert im Bereich von 3,5 bis 5,5 bevorzugt im Bereich von 4,0 bis 5,0 aufweist.

Weiterhin ist es vorteilhaft, wenn als kosmetischer Träger Wasser enthalten ist, wobei Wasser vorteilhaft in einem Anteil von 50 bis 95 Gew.-%, bezogen auf das Gesamtgewicht der Zubereitung, enthalten ist.

Die Zubereitung der Erfindung ist ferner vorteilhaft dadurch gekennzeichnet, dass diese eine Viskosität im Bereich von 6000 bis 15000 mPa·s bevorzugt im Bereich von 8000 bis 12000 mPa·s aufweist.

### Beispiele

Nachfolgende Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitung bezogen.

Bsp.3 ist erfindungsgemäß. Bsp.1 und Bsp.2 sind Vergleichsbeispiele.

| **INCI/Inhaltstoffe** | **Bsp.1** | **Bsp.2** | **Bsp.3** | |
|---|---|---|---|---|
| Aqua | ad 100 | ad 100 | ad 100 | |
| Glycerin | 3 | 3 | 3 | |
| Ceratonia Siliqua Gum | 0,5 | 0,5 | 0,5 | |
| Cetearyl Alcohol | 3,5 | 3,5 | 3,5 | |
| Polyglyceryl-10 Stearate | 2 | 2 | 2 | |
| Caprylic/Capric Triglyceride | 7 | 7 | 7 | |
| Phenoxyethanol | 0,8 | 0,8 | 0,8 | |
| Alcohol Denat. | 5 | 5 | 5 | |
| Epsilon-Polylysine | 0,7 | | 0,2 | |
| Propanediol Caprylate | | 0,7 | 0,5 | |
| Citric Acid | 0,4 | 0,05 | 0,1 | |
| | | | | |
| pH-Wert | 4,45 | 4,85 | 4,8 | |
| Viskosität (mPa·s) | 10900 | 11050 | 9900 | |
| | | | | |

| **Ergebnisse Suspensions-test nach angegebener Zeit (in colony forming units)** | | | | |
|---|---|---|---|---|
| **C. Acnes** | | | | Unbehandelte Kontrolle |
| 0-Wert* | 8,22E+06 | 8,22E+06 | 8,22E+06 | 8,22E+06 |
| 20 min* | 8,61E+06 | 1,21E+07 | 5,69E+06 | 8,22E+06 |
| 60 min* | 5,39E+06 | 1,05E+07 | 2,56E+05 | 7,32E+06 |
| 180 min* | 2,42E+06 | 9,57E+06 | 4,70E+03 | 8,25E+06 |

| | | | | |
|---|---|---|---|---|
| *Angaben in Colony Forming Units (CFU) | | | | |

Die antimikrobielle Wirksamkeit der erfindungsgemäßen Zubereitung gegen *Propionibakterium acnes (P. acnes)* wurde zum Vergleich mit verschiedenen Formulierungen untersucht.

Dazu wurde das Bakterium P. *acnes (DSM 1897)* aus einem nach EN 12353 angelegten Cryoröhrchen auf einer entsprechenden Agarplatte (Cost-Agar) ausgestrichen und bei 37°C 5 Tage unter anaeroben Bedingungen bebrütet.

Eine Impföse der Bakterien wurde zu 10mL anaerobem Medium gegeben und im Impedanzgerät mit dem Programm PR37NT unter anaeroben Bedingungen inkubiert. Anschließend wurde diese Bakteriensuspension auf eine OD (optische Dichte) von 0,3-0,5 eingestellt. Die eingestellte Bakteriensuspension wurde dann 1:10 mit anaerobem Medium verdünnt, um eine Keimzahl von ungefähr 5*10⁶ zu gewährleisten. Diese Bakteriensuspension wurde anschließend für den Test eingesetzt.

Der Suspensionstest wurde per Hand durchgeführt. Hierzu wurden 500µL der zu testenden Wirkstofflösungen bzw. im Falle der Kontrollen 500 µl anaerobem Medium in Mikroreaktionsgefäßen vorgelegt. Anschließend wurden 500 µl der Bakteriensuspension hinzugegeben und gemischt. Zum Zeitpunkt t₀ wurde eine Probe aus den Kontrollen entnommen. Die Mikroreaktionsgefäße wurden nun im Thermoschüttler bei 37 °C inkubiert. Nach 20 min, 60 min und 180 min bei 37°C wurden aus den Mikroreaktionsgefäßen im Thermoschüttler jeweils 100µL entnommen und zu 900µL Whitley Anaeroben Medium pipettiert (10⁻¹-Verdünnung). Diese Verdünnungen wurden mittels Spiralplatter auf Agarplatten ausplattiert. Eine Ausnahme stellt hier die Kontrolle dar, die nochmal 1:100 verdünnt wurde (10⁻³-Verdünnug). Bei den Kontrollen wurden beide Verdünnungen ausplattiert, einschließlich des Zeitpunkts T=0 Minuten. Zum Schluss wurden die Platten bei 37°C 5 Tage unter anaeroben Bedingungen bebrütet und die Zellzahl bestimmt.

Aus der Anzahl der bestimmten Colony Forming Units (CFU) für c. acnes ist zu entnehmen, dass nur die erfindungsgemäße Kombination zu einer Wirksamen Reduktion der Bakterienzahl führt.

| | **Bsp 4** | **Bsp 5** | **Bsp 6** | **Bsp 7** | **Bsp 8** |
|---|---|---|---|---|---|
| e-Polylysine | **0,1** | **0,4** | **0,3** | **0,5** | **0,7** |
| Caprylic/Capric Triglyceride | 5 | 5 | 5 | 5 | 5 |
| Polyglyceryl-10 Stearate | 2 | 0 | 0 | 0 | 2 |
| Polyglyceryl-3 Methylglucose Distearate | 0 | 2 | 0 | 0 | 0 |
| Polyglyceryl-6 Stearate | 0 | 0 | 1,8 | 1,8 | 0 |
| Polyglyceryl-6 Behenate | 0 | 0 | 0,2 | 0,2 | 0 |
| Tapioca Starch | 0 | 0 | 0 | 2,1 | 0 |
| Glycerin | 3 | 6 | 6 | 3 | 3 |
| Propanediol Caprylate | 0,6 | 0,4 | 0,6 | 0,2 | 0,1 |
| Citric Acid | 0,15 | 0,15 | 0,15 | 0,15 | 0,15 |
| Phenoxyethanol | 0,8 | 0,8 | 0,8 | 0,8 | 0,8 |
| Alcohol Denat | 5 | 5 | 5 | 5 | 5 |
| Cetearyl Alcohol | 3 | 3,5 | 3,5 | 3,5 | 3,5 |
| Stearyl Alcohol | 0,5 | 0 | 0 | 0 | 0 |
| Ceratonia Siliqua Gum | 0,5 | 0,5 | 0,5 | 0,5 | 0,5 |
| Hydroxypropyl Guar | 0 | 0 | 0 | 0 | 0,2 |
| Aqua | Ad 100 | Ad 100 | Ad 100 | Ad 100 | Ad 100 |

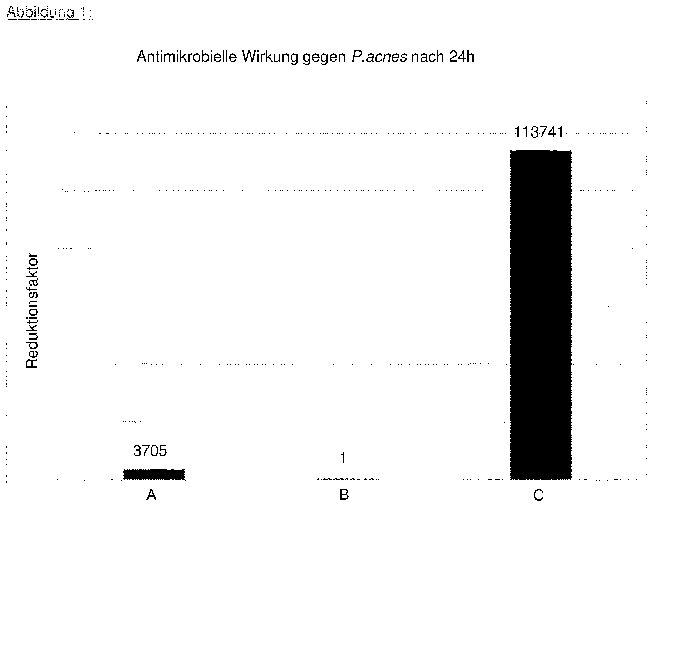

## Patentansprüche

1. Wirkstoffkombination umfassend ein Polylysin und Propanediol Caprylate.

2. Wirkstoffkombination nach Anspruch 1 **dadurch gekennzeichnet, dass** das Gewichtsverhältnis von Polylysin zu Propanediol Caprylate von 10:1 bis 1:10, bevorzugt von 5:1 bis 1:8, bevorzugt von 1:1 bis 1:5 und insbesondere bevorzugt von 1:2 bis 1:3 beträgt.

3. Wirkstoffkombination nach einem der vorhergehenden Ansprüche **dadurch gekennzeichnet, dass** ε-Polylysin in der Wirkstoffkombination enthalten ist.

4. Wirkstoffkombination nach einem der vorhergehenden Ansprüche **dadurch gekennzeichnet, dass** das Gewichtsverhältnis von ε-Polylysin zu Propanediol Caprylate von 10:1 bis 1:10, bevorzugt von 5:1 bis 1:8, bevorzugt von 1:1 bis 1:5 und insbesondere bevorzugt von 1:2 bis 1:3 beträgt.

5. Wirkstoffkombination gemäß einem der vorhergehenden Ansprüche zur Verwendung bei der Behandlung von Acne auf der menschlichen Haut.

6. Kosmetische und/oder dermatologische Zubereitung umfassend eine Wirkstoffkombination gemäß einem der Ansprüche 1 - 4.

7. Zubereitung nach Anspruch 6 **dadurch gekennzeichnet, dass** der Anteil von Polylysin von 0,01 bis 1 Gew.-%, bevorzugt von 0,1 bis 0,5 Gew.-% und insbesondere bevorzugt von 0,15 bis 0,3 Gew.-% beträgt, jeweils bezogen auf die Zubereitung.

8. Zubereitung nach einem der Ansprüche 6 oder 7 **dadurch gekennzeichnet, dass** ε-Polylysin enthalten ist.

9. Zubereitung nach einem der Ansprüche 6 bis 8 **dadurch gekennzeichnet, dass** der Anteil von ε-Polylysin von 0,01 bis 1 Gew.-%, bevorzugt von 0,1 bis 0,5 Gew.-% und insbesondere bevorzugt von 0,15 bis 0,3 Gew.-% beträgt, jeweils bezogen auf die Zubereitung.

10. Zubereitung nach einem der Ansprüche 6 bis 9 **dadurch gekennzeichnet, dass** der Anteil von Propanediol Caprylate von 0,01 bis 3 Gew.-%, bevorzugt von 0,1 bis 1,5 Gew.-% und insbesondere bevorzugt von 0,2 bis 0,8 Gew.-% beträgt, jeweils bezogen auf die Zubereitung.

11. Zubereitung nach einem der Ansprüche 6 bis 9 **dadurch gekennzeichnet, dass** eine Ölphase und eine wässrige Phase enthalten sind, wobei der Anteil der flüssigen Öle in der Zubereitung von 5 bis 15 Gew.-%, bevorzugt von 6 bis 11 Gew.-% und insbesondere bevorzugt von 6,5 bis 10 Gew.-%, bezogen auf das Gesamtgewicht der Zubereitung, beträgt.

12. Zubereitung gemäß einem der Ansprüche 6 bis 11 zur Verwendung bei der Prophylaxe und/oder Behandlung von Akne.

13. Wirkstoffkombination gemäß einem der Ansprüche 1 bis 4 zur Verwendung bei der Prophylaxe und/oder Behandlung von Akne.

## Claims

1. Active-ingredient combination comprising a polylysine and propanediol caprylate.

2. Active-ingredient combination according to Claim 1, **characterized in that** the ratio by weight of polylysine to propanediol caprylate is from 10:1 to 1:10, preferably from 5:1 to 1:8, preferably from 1:1 to 1:5 and particularly preferably from 1:2 to 1:3.

3. Active-ingredient combination according to either of the preceding claims, **characterized in that** ε-polylysine is present in the active-ingredient combination.

4. Active-ingredient combination according to any of the preceding claims, **characterized in that** the ratio by weight of ε-polylysine to propanediol caprylate is from 10:1 to 1:10, preferably from 5:1 to 1:8, preferably from 1:1 to 1:5 and particularly preferably from 1:2 to 1:3.

5. Active-ingredient combination according to any of the preceding claims for use in the treatment of acne on human skin.

6. Cosmetic and/or dermatological preparation comprising an active-ingredient combination according to any of Claims 1 - 4.

7. Preparation according to Claim 6, **characterized in that** the proportion of polylysine is from 0.01% to 1% by weight, preferably from 0.1% to 0.5% by weight and particularly preferably from 0.15% to 0.3% by weight, based in each case on the preparation.

8. Preparation according to either of Claims 6 and 7, **characterized in that** ε-polylysine is present.

9. Preparation according to any of Claims 6 to 8, **characterized in that** the proportion of ε-polylysine is from 0.01% to 1% by weight, preferably from 0.1% to 0.5% by weight and particularly preferably from 0.15% to 0.3% by weight, based in each case on the preparation.

10. Preparation according to any of Claims 6 to 9, **characterized in that** the proportion of propanediol caprylate is from 0.01% to 3% by weight, preferably from 0.1% to 1.5% by weight and particularly preferably from 0.2% to 0.8% by weight, based in each case on the preparation.

11. Preparation according to any of Claims 6 to 9, **characterized in that** an oil phase and an aqueous phase are present, the proportion of the liquid oils in the preparation being from 5% to 15% by weight, preferably from 6% to 11% by weight and particularly preferably from 6.5% to 10% by weight, based on the total weight of the preparation.

12. Preparation according to any of Claims 6 to 11 for use in the prophylaxis and/or treatment of acne.

13. Active-ingredient combination according to any of Claims 1 to 4 for use in the prophylaxis and/or treatment of acne.

## Revendications

1. Combinaison de principes actifs comprenant une polylysine et du caprylate de propanediol.

2. Combinaison de principes actifs selon la revendication 1, **caractérisée en ce que** le rapport en poids de polylysine à caprylate de propanediol est de 10:1 à 1:10, de préférence de 5:1 à 1:8, de préférence de 1:1 à 1:5 et en particulier de préférence de 1:2 à 1:3.

3. Combinaison de principes actifs selon l'une des revendications précédentes, **caractérisée en ce que** la combinaison de principes actifs contient de l'ε-polylysine.

4. Combinaison de principes actifs selon l'une des revendications précédentes, **caractérisée en ce que** le rapport en poids d'ε-polylysine à caprylate de propanediol est de 10:1 à 1:10, de préférence de 5:1 à 1:8, de préférence de 1:1 à 1:5 et en particulier de préférence de 1:2 à 1:3.

5. Combinaison de principes actifs selon l'une des revendications précédentes pour une utilisation lors du traitement de l'acné sur la peau humaine.

6. Préparation cosmétique et/ou dermatologique comprenant une combinaison de principes actifs selon l'une des revendications 1 - 4.

7. Préparation selon la revendication 6, **caractérisée en ce que** la proportion de polylysine est de 0,01 à 1% en poids, de préférence de 0,1 à 0,5% en poids et en particulier de préférence de 0,15 à 0,3% en poids, à chaque fois par rapport à la préparation.

8. Préparation selon l'une des revendications 6 à 7, **caractérisée en ce qu'**elle contient de l'ε-polylysine.

9. Préparation selon l'une des revendications 6 à 8, **caractérisée en ce que** la proportion d'ε-polylysine est de 0,01 à 1% en poids, de préférence de 0,1 à 0,5% en poids et en particulier de préférence de 0,15 à 0,3% en poids, à chaque fois par rapport à la préparation.

10. Préparation selon l'une des revendications 6 à 9, **caractérisée en ce que** la proportion de caprylate de propanediol est de 0,01 à 3% en poids, de préférence de 0,1 à 1,5% en poids et en particulier de préférence de 0,2 à 0,8% en poids, à chaque fois par rapport à la préparation.

11. Préparation selon l'une des revendications 6 à 9, **caractérisée en ce qu'**elle contient une phase huileuse et une phase aqueuse, la proportion des huiles liquides dans la préparation étant de 5 à 15% en poids, de préférence de 6 à 11% en poids et en particulier de préférence de 6,5 à 10% en poids, par rapport au poids total de la préparation.

12. Préparation selon l'une des revendications 6 à 11 pour une utilisation lors de la prophylaxie et/ou du traitement de l'acné.

13. Combinaison de principes actifs selon l'une des revendications 1 à 4 pour une utilisation lors de la prophylaxie et/ou du traitement de l'acné.
